# EUROPEAN PATENT SPECIFICATION

(11) **EP 3 375 472 B1**
(45) Date of publication and mention of the grant of the patent: **27.01.2021**
(21) Application number: 17161089.2
(22) Date of filing: 15.03.2017
(51) Int. Cl.: A61M 39/20, A61M 5/30, A61M 5/32, A61M 39/16

(54) **PEN-LIKE PORTABLE DEVICE FOR CLEANING NEEDLE-FREE IV-CONNECTORS**
STIFTARTIGE TRAGBARE VORRICHTUNG ZUR REINIGUNG NADELLOSER IV-VERBINDER
DISPOSITIF PORTATIF DE TYPE STYLO POUR LE NETTOYAGE DE CONNECTEURS IV SANS AIGUILLE

(43) Date of publication of application: 19.09.2018
(73) Proprietor: Asset Medikal Tasarim Sanayi ve Ticaret A.S., Basaksehir-Istanbul (TR)
(72) Inventor: TÜYSÜZ, Mehmet, Istanbul (TR)
(74) Representative: Sevinç, Erkan

(56) References cited:
- US-A1- 2009 028 750
- US-A1- 2011 044 850
- US-A1- 2016 144 118
- US-B2- 8 777 504

## Description

### Technical field of the invention

The present invention relates to an assembly of caps for protecting and cleaning needle-free intravenous connectors.

### Background of the invention

Devices for protecting, cleaning or disinfection of surfaces of needleless intravenous (iv) connectors are known and widely used in medicine.

A user, e.g. a health professional working at a hospital, uses this device in cleaning of an inlet tip of an iv-connector by rubbing the tip for a time of about 30 seconds or longer. The same tedious procedure is generally repeated multiple times by the same user in cleaning tens of different iv-connectors.

Said devices are disposable and should be immediately used once opened. Generally, these devices are present in separate units, or some versions of them are present in plurality attached together to form a mainly flat assembly which is not compact. Neither of said versions is suitable to be carried in a pocket of a garment, e.g. along with pens.

US20110044850A and US20080019889A provide examples to such devices, which include a cavity covered with a foil seal. US 8777504 B2, US 2013/0199947 A1, US 2009/0028750 A1 and US 8647308 B2 also provide further examples to the state of the art. US20160144118A discloses an assembly comprising a plurality of caps for being removably attached to a septum of a needle-free intravascular connector. Each cap in the assembly has a cavity, which is provided with a breakable sealing in order to prevent contamination until being used.

### Objects of the invention

Primary object of the present invention is to eliminate the above-mentioned shortcomings in the prior art.

Another object of the present invention is to provide an assembly of caps which are easy to use.

A further object of the present invention is to provide an assembly of caps with minimized costs.

An even further object of the present invention is to provide a cap enabling such assembly.

### Summary of the invention

The present invention is defined in claim 1. Advantageous embodiments of the invention are set out in dependent claims 2-9.
A cap is proposed for being removably attached to a septum of a needle-free intravascular connector, the cap comprising a protrusion at a first end, and a cavity at a second end distal to the first end; the protrusion having a circumferential side surface, and the cavity having a circumferential side surface with a form corresponding that of the protrusion and to fit the side surface of such protrusion, wherein the side surfaces of the protrusion and of the cavity have conjugate reversible fluid-tight fastening means, such that the cavity is hermetically sealable by introduction and fastening of an identical protrusion of a further cap thereinto. The present invention proposes an assembly comprising such caps.

### Brief explanation of the figures

The figures, brief explanations on which are herewith provided, are solely intended for providing a better understanding of the present invention and are as such not intended to define the scope of protection or the context in which said scope is to be interpreted in the absence of the description.

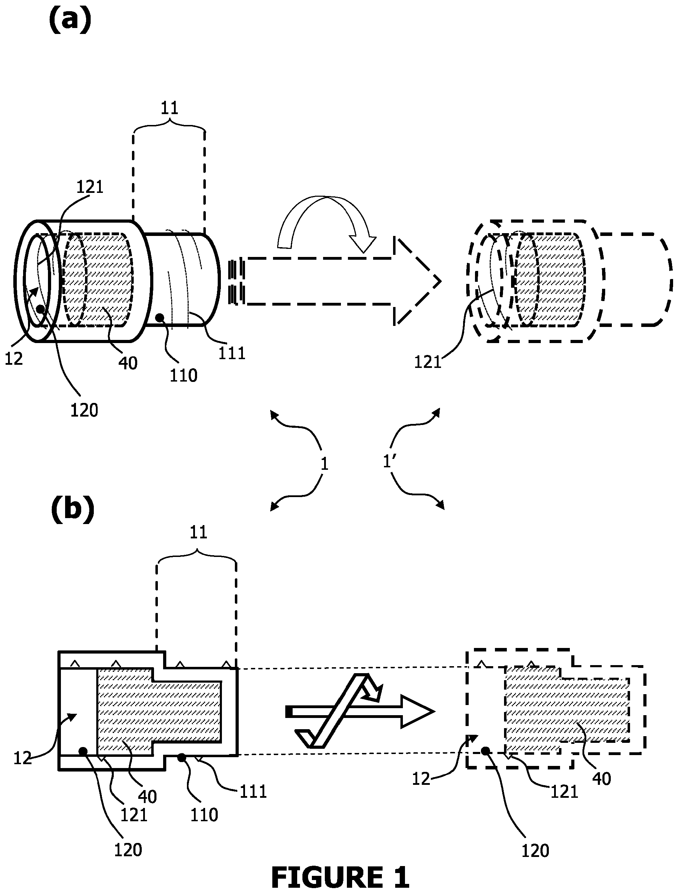

Figure 1 shows a schematic perspective view (a) demonstrating the fastening of two caps according to a first embodiment according to the present invention together, and (b) the same with longitudinal section views of said caps.

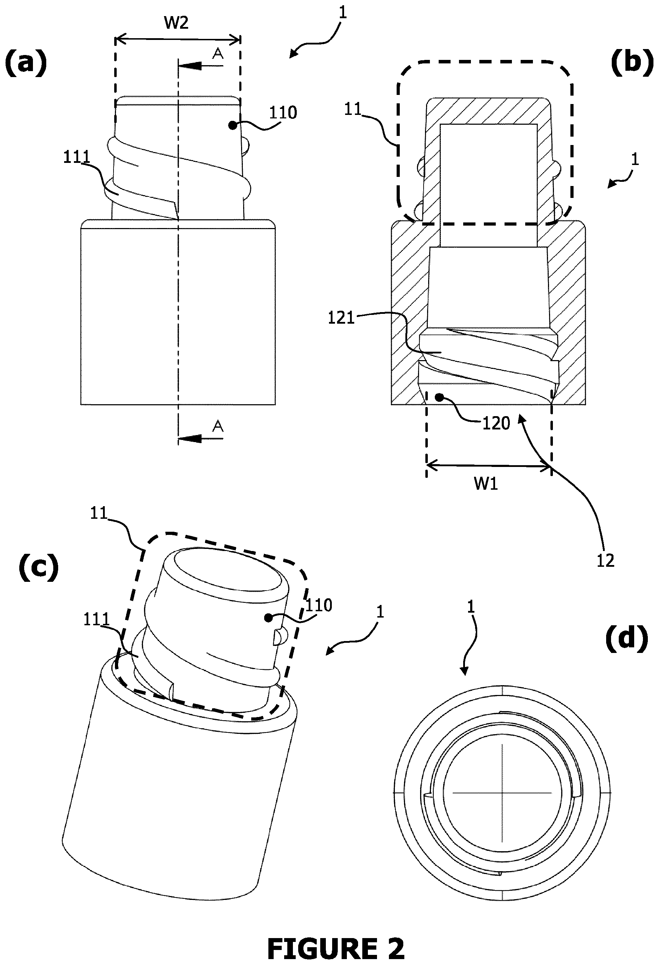

Figure 2 shows (a) a side view of the first embodiment according to the present invention, (b) a A-A longitudinal section view of the same, (c) a perspective view emphasizing the protrusion of the same, and (d) a top view from the cap side of the same.
Figure 3 (a) shows a side view of an assembly comprising a plurality of the caps shown in Fig. 2, and (b) B-B longitudinal section of the same.
Figure 4 shows a perspective view of the assembly shown in Fig. 3, wherein the clip is placed in an alternative direction and on the other end of the assembly.

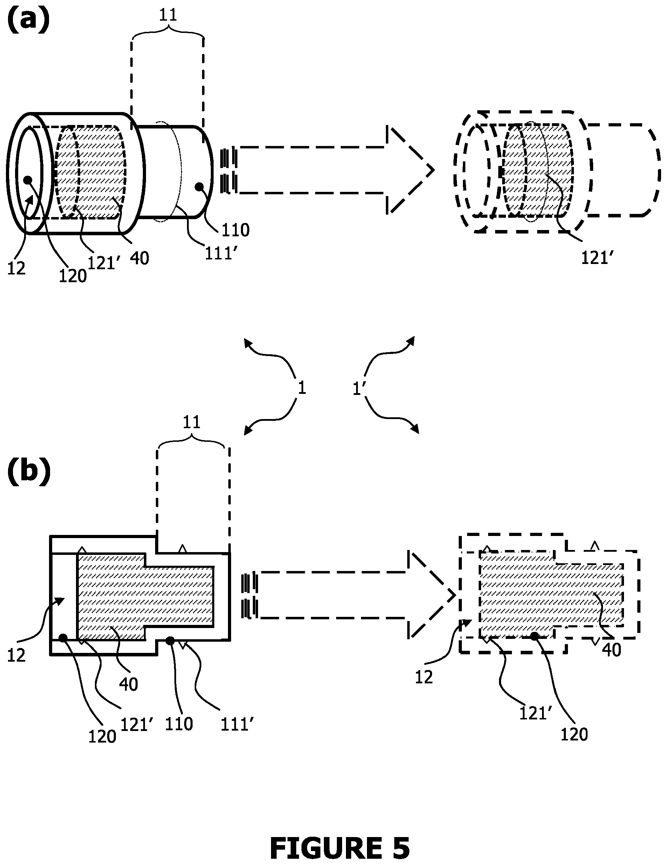

Figure 5 shows a schematic perspective view (a) demonstrating the fastening of two caps according to a second embodiment according to the present invention together, and (b) the same with longitudinal section views of said caps.
Figure 6 shows (a) a side view of the first embodiment according to the present invention, (b) a C-C longitudinal section view of the same, (c) a perspective view emphasizing the protrusion of the same, and (d) a top view from the cap side of the same.
Figure 7 (a) shows a side view of an assembly comprising a plurality of the caps shown in Fig. 6, and (b) D-D longitudinal section of the same.

### Detailed description of the invention

With reference to the figures briefly described above, the present invention proposes a cap (1) for being removably attached to a septum of a needle-free intravascular connector. The cap (1) comprises a protrusion (11) at a first end, and a cavity (12) at a second end distal to said first end. The protrusion (11) has a circumferential side surface (110). The cavity has a circumferential side surface (120). The side surface (120) of the cavity (12) has a form which corresponds to the side surface (110) of the protrusion (11) and thus the form of the side surface (120) of the cavity (12) fits the side surface (110) of such protrusion (11).

The side surface (110) of the protrusion (11) and the side surface (120) of the cavity (12) have conjugate reversible fluid-tight fastening means (111, to engage with 121 or 111' to engage with 121'), such that the cavity (12) is hermetically sealable by introduction and fastening of a protrusion (11) of a further cap (1') thereinto wherein the protrusion (11) of the further cap (1') is identical to the protrusion (11) of the cap (1) according to the present invention.

The above-specified properties of the side surfaces of the protrusion (11) and that of the cavity (12) enables hermetical fastening between the side surface (120) of the cavity (12) and a protrusion (11) of a further and identical cap (1'), thus it enables prevention of any fluid flow between the cavity (12) and the surroundings when a protrusion (11) of a further and identical cap (1') is mounted (i.e. reversibly fitted) into the cavity (12) through the conjugate fluid-tight reversible fastening means.

The fluid-tight fitting can be further enhanced by selecting the material of which the side surfaces of the protrusion and cavity in accordance with a liquid intended to be placed into the cavity: if said liquid is hydrophilic, the material can be selected from rather non-polar materials; and if said is hydrophobic, said material can be selected from polar materials. Lists of polar (hydrophilic) and non-polar (hydrophobic) materials (e.g. such polymeric materials or their mixtures) are available in sources related to materials science or chemistry.

The suitability of the cap (1) for being attached to a septum of an iv-connector is easily achievable by arranging the dimensions of the cavity. The width (W1) of the cavity in a direction perpendicular to that of introduction of a septum, can be substantially equal to or slightly greater than a standard connector tip (which includes the septum), e.g. a luer connector or a needleless adapter, examples of which being known in the art.

Said conjugate fastening means can be screw threads (111 and 121). This embodiment is suitable for cleaning and protecting a septum of an iv-connector with threaded tip (such as a needleless adapter). In an alternative embodiment, the conjugate fastening means can include a groove (121') around one of the side surfaces (110 or 120) and a corresponding dentation (111') around the other side surface (120 or 110, respectively for the embodiment mentioned in this sentence); arranged, shaped and sized to enable a reversible snap-fit connection between the fastening means on the side surface (120) of the cavity (12) and that on the side surface (110) of an identical protrusion (11) of a further cap (1'). This embodiment is suitable for cleaning and protecting a septum of an iv-connector with mainly cylindrical tip (such as a luer connector) which can have a circumferential groove or dentation.

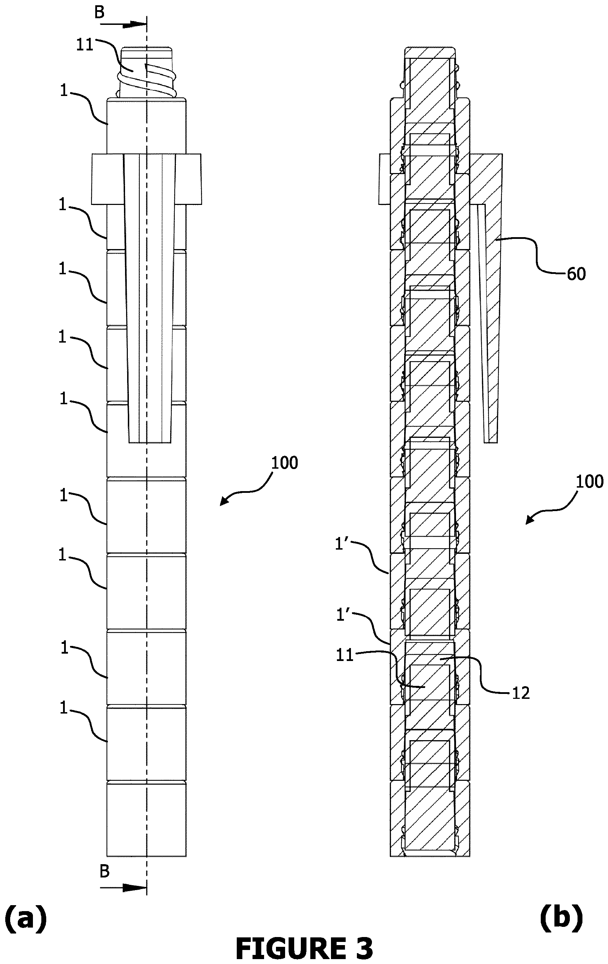

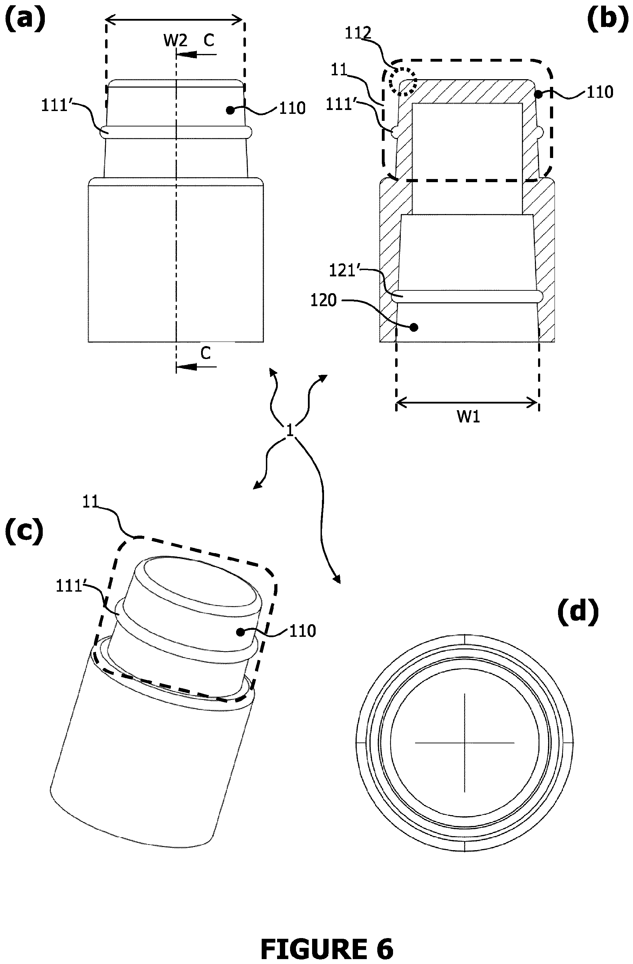

The cap (1) can be made of a resilient material (such as a flexible polymer). In this case, the width (W1, e.g. the diameter of an entry opening of the cavity) of the cavity (12) can be substantially equal to or smaller than the width (W2, e.g. the diameter of a tip of the protrusion) of the protrusion (11) perpendicular to a main central axis (A1) of the cavity (12). The main central axis (A1) of the cavity may coinside with a main central axis of the protrusion and thus define a main central axis also for the cap as depicted in Fig.6b, and accordingly, also a main central axis for an assembly of identical caps fastened or mounted together as depicted in Fig.3b. In such cap (1), the cavity (12) gets flexed outwards when a protrusion (11) of a further and identical cap (1') (i.e. an identical protrusion (11) of a further cap (1')) is pushed into the cavity (12), allowing introduction of the protrusion (11), resulting in an elevated pressure exerted by the cavity side surface (120) circumferentially onto the side surface (110) of the protrusion (11). Fluid transfer into or from the cavity (12) is thus even better prevented with higher values of said pressure. This feature is particularly useful for the embodiment at which snap-fit connection is enabled.

In a cap (1) according to the present invention, the protrusion (11) can have a rounded edge (112) distal to the cavity (12) of the same cap (1). This facilitates the introduction of the protrusion (11) into a cavity (12) of an identical cap (1'). Furthermore, in an embodiment where the cap (1) is made of a resilient material, width (W1) of the cavity (12) can be (at least slightly) smaller than that of the protrusion (11) perpendicular to the main central axis (A1) of the cavity, or perpendicular to direction of introduction of an identical protrusion (11) of a further cap (1') into the cavity. This feature is useful for both threaded and snap-fit type fastening means as described above, resulting in even higher pressure exerted by the cavity side surface (120) circumferentially onto the protrusion side surface (110).

The cap (1) can comprise a flexible and adsorbent material (40) placed into the cavity (12). This enables the cavity (12) to function as a liquids reservoir upon wetting or soaking of the adsorbent material (40) adapted for rubbing, with a liquid. In such cap (1), the size of the flexible and adsorbent material (40) can be arranged such that the fastening means on the side surface (120) of the cavity (12) is at least partly covered by the adsorbent material (40). In this embodiment, in case where the adsorbent material (40) is wetted or soaked with a liquid, the liquid can be partly squeezed out of the adsorbent material (40) merely by introduction of a septum of an iv-connector tip into the cavity, thereby achieving an enhanced propogation of the liquid on a frontal portion of the iv-connector including the septum, and also around the outer side surfaces of the tip of the iv-connector.

The adsorbent material (40) can be a synthetic foam material. Alternatively, the adsorbent material (40) can comprise natural fibers (e.g. cotton) or synthetic fibers. Various options of such adsorbent materials suitable for being soaked or wetted with liquids for cleaning, disinfecting or sterilization are available in various sources related art of medical supplies industry.

In a further embodiment according to the present invention, the adsorbent material (40) in the cap (1) is wetted or soaked with a liquid for cleaning, disinfecting or sterilizing a septum of a needle-free intravascular connector. Such liquids can comprise a cleaning agent or that mixed with a solvent. Suitable cleaning agents are already known in the related art, e.g. isopropyl alcohol, chlorhexidine, povidone-iodine, hydrogen peroxide, soap, and hydrochloric acid. With this embodiment, the cap (1) can be readily used in cleaning, disinfecting or sterilizing of an iv-connector upon its cavity (12) being released from a further protrusion (11) identical with that on the same cap (1).

Accordingly, the present invention proposes an assembly comprising a plurality of caps (1) according to any of the above specifications. In the assembly, the cavity of one or more of the caps (1) is hermetically sealed by fastening of a protrusion introduced thereto through said fastening means.

In the assembly, one or more of the caps (1) with hermetically sealed cavities (12) can include a liquid for cleaning, disinfecting or sterilizing a septum of a needle-free intravascular connector. The hermetically sealed cavities of one or more of the caps (1) can comprise a flexible and adsorbent material (40) placed into said cavities (12), and they can be wetted or soaked with such liquid.

The assembly (100) enables prevention of any contamination of the cavity of a cap (1) in the assembly (100), from unwanted matter available outside the cavity (12), by maintaining the cavity (12) hermetically sealed by the protrusion (11) of another cap (1') which is mainly identical with that of the same cap (1) in the sense of the present invention; without necessitating any further removable sealing means.

The cap (1) and assembly (100) comprising the cap (1) according to the present invention minimizes the production steps for the cap (1), eliminating any seal provision step for covering a cavity (e.g. a cap or a tearable or flexible film for direct disposal without further use upon removal from the cavity opening), and further eliminating the material and processing costs related to provision of such seal.

A user (e.g. health professional) can easily pull or unscrew a cap (1) according to the present invention and thus unfasten the same e.g. from a protrusion (11) of a neighboring cap (1) from the assembly (100), and directly use the cap (1) in cleaning, disinfecting or sterilizing a septum or tip of an iv-connector, free from burden to remove any further sealing means. Thus, upon removal of a cap (1) from the assembly (100), its cavity (12) or any cleaning material (fluid or adsorbent material provided with fluid) is instantly exposed without requiring any further step. The caps (1) maintain their relative positions in the assembly (100) until being unfastened by user's will.

The assembly (100) can be further provided with a clip (60) for attachment of the assembly to a pocket on a garment, e.g. on a shirt or on a laboratory coat. The clip (60) can be arranged in vicinity of any of the ends of the assembly. The caps (1) can be conveniently dismounted (i.e. released, removed, taken) one by one from the distal end of the assembly (100) with respect to the clip (60).

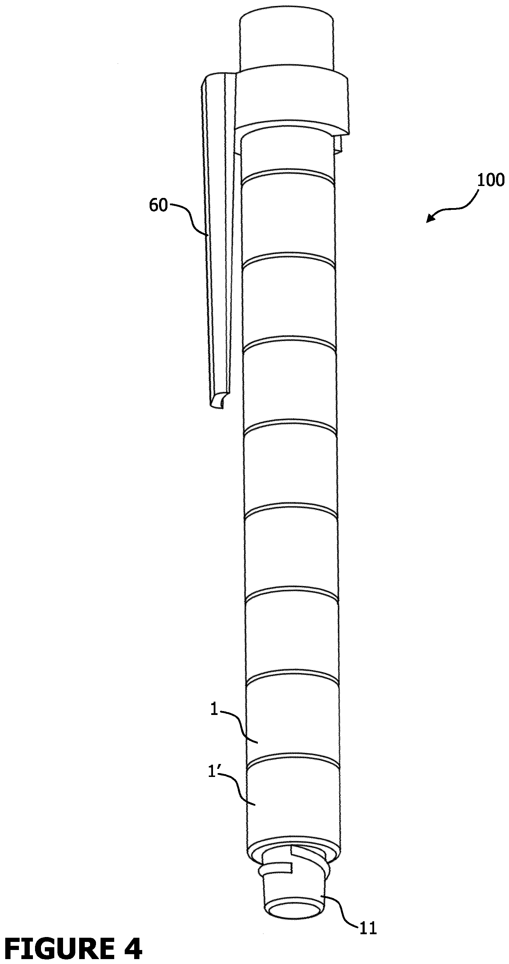

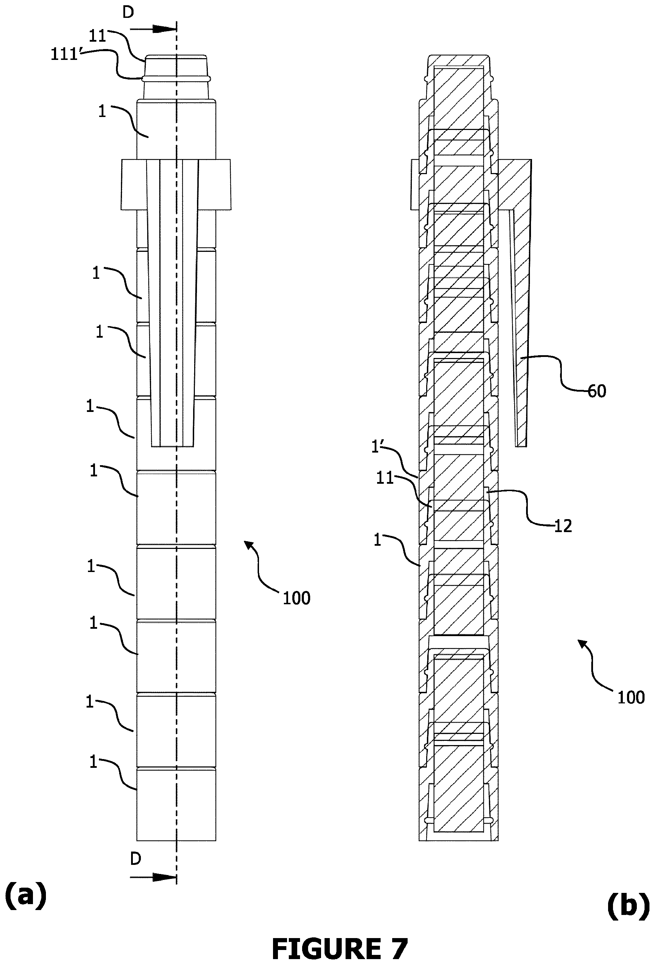

For instance, the clip (60) can be placed near an end where a protrusion (11) is exposed as shown in Figures 3 and 7.
Alternatively, the clip (60) can be placed near the other end distal to the exposed protrusion (11) as shown in an alternative given in Figure 4. The latter embodiment (shown in Fig. 4) can be considered even more preferable, since in this embodiment hand contact mainly occurs on the side surfaces of the cap (1), especially on the side surface (110) of the protrusion (11). This minimizes possible contamination around the cavity.

More importantly, in this embodiment, in case where a cap (1) on a tip of the assembly (100) is dismounted, the cavity (12) of said cap (1), cavity side surfaces (120) and/or any cleaning liquid in the cavity (i.e. the liquid itself or in an adsorbent material as described above) is exposed to the surroundings for the first time, eliminating any potential contamination of the cavity content and minimizing leaking or drying out of liquid in the cavity.

The assembly of caps according to the present invention also facilitate the work of the user by being compact. The user can keep the assembly of the caps (1) for instance in her pocket, and when necessary, she handle it with a first single move, and then dismount or release a cap (1) from the assembly at a second single move of unfastening it from a neighboring cap (1). The assembly maintains its compact and non-disperse form these advantages until all of the caps (1) with the hermetically sealed cavities (12) are used up.

Thus, the below objects are achieved by the present invention:
- elimination of the above-mentioned shortcomings in the prior art,
- provision of an assembly of caps which are easy to use,
- provision of an assembly of caps with minimized costs.

## Claims

1. An assembly (100) comprising a plurality of caps (1, 1') for being removably attached to a septum of a needle-free intravascular connector, wherein
one or more of said caps (1, 1') comprise a protrusion (11) at a first end, and a cavity (12) at a second end distal to the first end;
the protrusion (11) has a circumferential side surface (110), and
the cavity (12) has a circumferential side surface (120) with a form corresponding the side surface (110) of the protrusion (11) and to fit the side surface (110) of such protrusion (11);
the side surface (110) of the protrusion (11) and the side surface (120) of the cavity (12) have pre-formed conjugate reversible fluid-tight fastening means (111, 111' and 121, 121'), such that the cavity is hermetically sealable by introduction and fastening of an identical protrusion of a further cap thereinto, and
the conjugate fastening means are screw threads (111, 121), or
the conjugate fastening means are a groove (121) around one of the side surfaces (110 or 120) and a corresponding dentation around the other side surface (120 or 110), arranged to enable a reversible snap-fit connection between the fastening means on the side surface (120) of the cavity (12) and that on the side surface (110) of an identical protrusion (11) of a further cap (1');
further wherein the cavity (12) of one or more of the caps (1') is hermetically sealed by fastening of a protrusion (11) of another of the caps (1) introduced thereto through said fastening means (111, 111' and 121, 121').

2. The assembly according to the claim 1, wherein said one or more caps (1, 1') are made of a resilient material and the width (W1) of the cavity (12) is substantially equal to or smaller than the width (W2) of the protrusion (11) perpendicular to a main central axis of the cavity (12).

3. The assembly according to any of the claims 1 or 2, wherein said one or more caps (1, 1') comprise a flexible and adsorbent material (40) placed into the cavity (12).

4. The assembly according to the claim 3, wherein in said one or more caps (1, 1') the size of the flexible and adsorbent material (40) is arranged such that the fastening means (121) on the side surface (120) of the cavity (12) is at least partly covered by the adsorbent material (40).

5. The assembly according to any of the claims 3 or 4, wherein the adsorbent material (40) is a synthetic foam material.

6. The assembly according to any of the claims 3 or 4, wherein the adsorbent material (40) comprises natural or synthetic fibers.

7. The assembly according to any of the claims 3 to 6, wherein the adsorbent material (40) is wetted or soaked with a liquid for cleaning, disinfecting or sterilizing a septum of a needle-free intravascular connector.

8. The assembly according to any of the claims 1 to 7, wherein in said one or more caps (1, 1'), the protrusion (11) has a rounded edge (112) distal to the cavity (12).

9. The assembly according to any of claims 1 to 8, further provided with a clip (60) for attachment of the assembly (100) to a pocket on a garment.

## Patentansprüche

1. Anordnung (100), umfassend eine Mehrzahl von Kappen (1, 1') zum lösbaren Befestigen an einem Septum eines nadellosen intravaskulären Verbinders, wobei
eine oder mehrere der Kappen (1, 1') eine Vorwölbung (11) an einem ersten Ende und einen Hohlraum (12) an einem zweiten Ende distal zum ersten Ende umfassen;
die Vorwölbung (11) eine umlaufende Seitenfläche (110) aufweist, und
der Hohlraum (12) eine umlaufende Seitenfläche (120) mit einer Form entsprechend der Seitenfläche (110) der Vorwölbung (11) und zum Passen auf die Seitenfläche (110) einer solchen Vorwölbung (11) aufweist;
die Seitenfläche (110) der Vorwölbung (11) und die Seitenfläche (120) des Hohlraums (12) vorgeformte, konjugierte, umkehrbare, fluiddichte Befestigungsmittel (111, 111' und 121, 121') aufweisen, derart dass der Hohlraum durch Einführen und Befestigen einer identischen Vorwölbung einer weiteren Kappe darin hermetisch abdichtbar ist,
und
die konjugierten Befestigungsmittel Schraubengewinde (111, 121) sind, oder
die konjugierten Befestigungsmittel eine Nut (121) um eine der Seitenflächen (110 oder 120) und eine entsprechende Vertiefung um die andere Seitenfläche (120 oder 110) sind, die angeordnet sind, um eine reversible Schnappverbindung zwischen den Befestigungsmitteln an der Seitenfläche (120) des Hohlraums (12) und denjenigen an der Seitenfläche (110) einer identischen Vorwölbung (11) einer weiteren Kappe (1') zu ermöglichen;
wobei ferner der Hohlraum (12) von einer oder mehreren der Kappen (10) durch Befestigen einer Vorwölbung (11) einer anderen der Kappen (1), die durch die Befestigungsmittel (111, 111' und 121, 121') in diesen eingeführt ist, hermetisch abgedichtet ist.

2. Anordnung nach Anspruch 1, wobei die eine oder die mehreren Kappen (1, 1') aus einem elastischen Material hergestellt sind und die Breite (W1) des Hohlraums (12) im Wesentlichen gleich oder kleiner ist als die Breite (W2) der Vorwölbung (11) senkrecht zu einer zentralen Hauptachse des Hohlraums (12).

3. Anordnung nach einem der Ansprüche 1 oder 2, wobei die eine oder die mehreren Kappen (1, 1') ein in den Hohlraum (12) eingebrachtes flexibles und adsorbierendes Material (40) umfassen.

4. Anordnung nach Anspruch 3, wobei in der einen oder den mehreren Kappen (1, 1') die Größe des flexiblen und adsorbierenden Materials (40) so angeordnet ist, dass das Befestigungsmittel (121) auf der Seitenfläche (120) des Hohlraums (12) zumindest zum Teil durch das adsorbierende Material (40) bedeckt ist.

5. Anordnung nach einem der Ansprüche 3 oder 4, wobei das adsorbierende Material (40) ein synthetisches Schaumstoffmaterial ist.

6. Anordnung nach einem der Ansprüche 3 oder 4, wobei das adsorbierende Material (40) natürliche oder synthetische Fasern umfasst.

7. Anordnung nach einem der Ansprüche 3 bis 6, wobei das adsorbierende Material (40) mit einer Flüssigkeit zum Reinigen, Desinfizieren oder Sterilisieren eines Septums eines nadellosen intravaskulären Verbinders benetzt oder getränkt ist.

8. Anordnung nach einem der Ansprüche 1 bis 7, wobei in der einen oder den mehreren Kappen (1, 1') die Vorwölbung (11) einen abgerundeten Rand (112) distal zu dem Hohlraum (12) aufweist.

9. Anordnung nach einem der Ansprüche 1 bis 8, ferner mit einem Clip (60) zur Befestigung der Anordnung (100) an einer Tasche an einem Kleidungsstück bereitgestellt ist.

## Revendications

1. Ensemble (100) comprenant une pluralité de capuchons (1, 1') destinés à être fixés de manière amovible à un septum d'un connecteur intravasculaire sans aiguille,
un ou plusieurs desdits capuchons (1, 1') comprenant une saillie (11) à une première extrémité, et une cavité (12) à une seconde extrémité distale par rapport à la première extrémité ;
la saillie (11) ayant une surface latérale circonférentielle (110), et
la cavité (12) ayant une surface latérale circonférentielle (120) avec une forme correspondant à la surface latérale (110) de la saillie (11) et pour s'ajuster sur la surface latérale (110) de ladite saillie (11) ;
la surface latérale (110) de la saillie (11) et la surface latérale (120) de la cavité (12) ayant des moyens de fixation étanches aux fluides, réversibles conjugués, préformés (111, 111' et 121, 121'), de telle sorte que la cavité est apte à être scellée de manière hermétique par introduction et fixation d'une saillie identique d'un autre capuchon à l'intérieur de celle-ci, et
les moyens de fixation conjugués étant des filetages (111, 121), ou
les moyens de fixation conjugués étant une gorge (121) autour de l'une des surfaces latérales (110 ou 120) et une dent correspondante autour de l'autre surface latérale (120 ou 110), agencée pour permettre une liaison par emboîtage élastique réversible entre le moyen de fixation sur la surface latérale (120) de la cavité (12) et celui sur la surface latérale (110) d'une saillie identique (11) d'un autre capuchon (1') ;
en outre, la cavité (12) de l'un ou de plusieurs des capuchons (1') étant scellée de manière hermétique par fixation d'une saillie (11) d'un autre des capuchons (1), introduite à l'intérieur de celle-ci, par l'intermédiaire desdits moyens de fixation (111, 111' et 121, 121').

2. Ensemble selon la revendication 1, dans lequel ledit ou lesdits capuchons (1, 1') sont faits d'une matière élastique et la largeur (W1) de la cavité (12) est sensiblement inférieure ou égale à la largeur (W2) de la saillie (11) perpendiculairement à un axe central principal de la cavité (12).

3. Ensemble selon l'une quelconque des revendications 1 ou 2, dans lequel ledit ou lesdits capuchons (1, 1') comprennent une matière souple et adsorbante (40) placée dans la cavité (12).

4. Ensemble selon la revendication 3, dans lequel, dans ledit ou lesdits capuchons (1, 1'), la taille de la matière souple et adsorbante (40) est agencée de telle sorte que le moyen de fixation (121) sur la surface latérale (120) de la cavité (12) est au moins partiellement couvert par la matière adsorbante (40).

5. Ensemble selon l'une quelconque des revendications 3 ou 4, dans lequel la matière adsorbante (40) est une matière mousse synthétique.

6. Ensemble selon l'une quelconque des revendications 3 ou 4, dans lequel la matière adsorbante (40) comprend des fibres naturelles ou synthétiques.

7. Ensemble selon l'une quelconque des revendications 3 à 6, dans lequel la matière adsorbante (40) est mouillée ou imprégnée avec un liquide pour nettoyer, désinfecter ou stériliser un septum d'un connecteur intravasculaire sans aiguille.

8. Ensemble selon l'une quelconque des revendications 1 à 7, dans lequel, dans ledit ou lesdits capuchons (1, 1'), la saillie (11) a un bord arrondi (112) distal par rapport à la cavité (12).

9. Ensemble selon l'une quelconque des revendications 1 à 8, comportant en outre une attache (60) pour fixation de l'ensemble (100) à une poche sur un vêtement.
